(19) Europäisches Patentamt
European Patent Office
Office européen des brevets

(11) Publication number: **0 178 708**
**B1**

(12) # EUROPEAN PATENT SPECIFICATION

(45) Date of publication of patent specification: **08.11.89**

(21) Application number: **85201529.6**

(22) Date of filing: **24.09.85**

(51) Int. Cl.$^4$: **C 07 D 235/10**, C 07 D 235/22, C 07 D 235/06, C 07 D 235/08, C 07 D 235/28, C 07 D 235/30, C 07 D 249/18, C 07 D 263/56, C 07 D 263/58, C 07 D 277/64, C 07 D 285/14

(54) **Heteroaromatic ether herbicides.**

(30) Priority: **18.10.84 GB 8426367**

(43) Date of publication of application:
**23.04.86 Bulletin 86/17**

(45) Publication of the grant of the patent:
**08.11.89 Bulletin 89/45**

(84) Designated Contracting States:
**AT BE CH DE FR GB IT LI LU NL SE**

(56) References cited:
**EP-A-0 023 022**
**EP-A-0 108 908**
**DE-A-1 542 868**
**DE-A-1 695 786**
**DE-A-2 815 621**
**DE-C-1 124 499**
**DE-C-1 232 922**
**FR-A-2 258 179**
**GB-A-1 087 101**
**LU-A- 77 123**
**US-A-3 472 935**
**US-A-4 299 965**

**A.R.KATRITZKY et al.: "Chemistry of the heterocyclic N-oxides", Academic Press, 1971, London (GB); pp. 130-131**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Clark, Michael Thomas**
**Torcross Wises Lane Borden**
**Sittingbourne Kent (GB)**
Inventor: **Munro, David**
**40 Harvesters Way**
**Maidstone Kent (GB)**
Inventor: **Gilmore, Ian James**
**32 Waterloo Road**
**Sittingbourne Kent (GB)**

(74) Representative: **Hunter, Keith Roger Ian et al**
**4 York Road**
**London SE1 7NA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 178 708 B1

**Description**

This invention relates to certain heteroaromatic diphenyl ether derivatives, the preparation of such compounds, herbicidal compositions containing them, and to their use in combating undesired plant growth.

Certain diphenyl ethers are known to be effective herbicides, for example UK Patent Application No. 2049 695 describes diphenyl ether ketone oximes and their use as herbicides. It has now been found that useful herbicidal activity is present in ethers bearing a bicyclic heteroaromatic group. Several of these ethers are known compounds.

French patent application publication number FR—A—2258179 discloses the use of certain 2-trifluoromethylbenzimidazole derivatives as anthelminthics. Luxembourg patent application number LU—A—77123 and West German patent application number DE—A—2815621 also disclose certain benzimidazole derivatives an anthelminthics.

U.S. patent number 3,472,935 discloses certain 2-dihalomethyl benzothaizoles and their use as nematicides.

West German patent application number DE—A—1,542,868 dicloses certain 2-trifluoromethyl and 2-pentafluoroethyl benzimidazole derivatives as herbicides, insecticides, molluscicides and fungicides.

British patent specification number 1,087,101 discloses certain 2-trichloromethyl benzoxazoles as biological toxicants, e.g., herbicides and fungicides.

Accordingly, the present invention provides diphenyl ethers having the general formula I:—

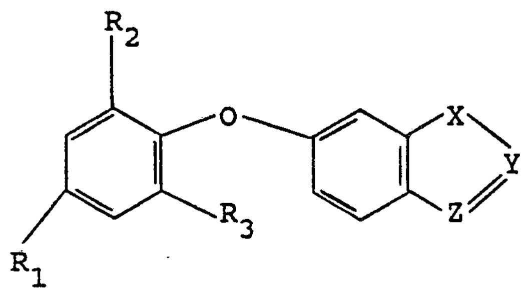

wherein

$R_1$ represents a haloalkyl, preferably trifluoromethyl, group;

$R_2$ and $R_3$, which may be the same or different, each independently represents a hydrogen or halogen, preferably chlorine, atom or a nitro, or haloalkyl, preferably trifluoromethyl, group;

one of Y and Z represents a nitrogen atom or an oxide thereof and the other represents a nitrogen atom or an oxide thereof or the group C—$R_4$;

X represents an oxygen or sulphur atom, a carbonyl, thiocarbonyl or sulphonyl group, or a group N—$R_5$ or $C(R_4)_2$;

$R_4$ represents a hydrogen atom or a mercaptan, hydroxyl, alkoxy, alkylthio, alkylsulphonyl, alkyl, amino, alkylureido, alkylcarbonyloxyalkyl, alkoxycarbonylalkylthio, or alkylcarbamoyl group, provided that when two groups $R_4$ are attached to the same carbon atom, then the said groups are not both hydroxyl, mercaptan or alkylthio; and

$R_5$ represents a hydrogen atom or a hydroxyl, alkoxy, alkylsulphonyl, alkyl, haloalkyl, amino, alkylureido, alkylcarbonyloxyalkyl or alkylcarbamoyl group, provided than when $R_1$ represents a trifluoromethyl group, $R_2$ represents a chlorine atom, $R_3$ represents a hydrogen atom, X represents N—$CH_3$ and Z represents N→O, Y may additionally represent C—CH=$NCH_3$.

When any of the substituents represents or contains an alkyl, or haloalkyl group, this contains up to 6, preferably up to 4, carbon atoms, methyl usually being the alkyl group of choice, and the halogen atom in the haloalkyl group is suitably fluorine or chlorine, with trifluoromethyl being the preferred alkyl group.

Preferred compounds are those wherein $R_1$ represents a trifluoromethyl group, $R_2$ represents a halogen, especially chlorine, atom, or a nitro group, and $R_3$ represents a hydrogen atom.

Z preferably represents a nitrogen atom, or an oxide thereof, and X preferably represents an oxygen or sulphur atom, or the group $NR_5$ in which $R_5$ represents a hydrogen atom or an alkyl, especially methyl, or alkylcarbamoyl, especially, methylcarbamoyl, group. Y preferably represents a nitrogen atom or the group $CR_4$ in which $R_4$ represents a hydrogen atom or mercaptan, methylthio, methylsulphonyl, methoxy, methyl, ethoxycarbonylethylthio, methylcarbonyloxymethyl, amino or methylureido group.

The invention also provides a process for the preparation of diphenyl ethers as defined above, which comprises cyclization of a compound of formula II

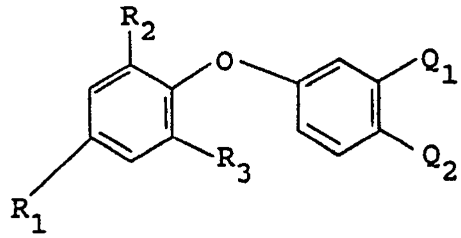

II

conditions/reagents require to effect cyclization are dependent on the component heteroatoms required in the resulting XYZ heterocyclic ring.

When the desired product is a thiadiazole, i.e. X is sulphur and Y and Z are both nitrogen atom, then a suitable starting material of formula II is that wherein $Q_1$ represents a mercapto group protected by, for example, a benzyl group, and $Q_2$ represents an amino group. Cyclization to the desired thiadiazole ring is suitably effected by reaction with an aqueous solution of an alkali metal nitrite, conveniently sodium nitrite, in the presence of an acid, conveniently a mineral acid such as sulphuric acid, at a temperature not exceeding 10°C. The starting material may be prepared from a compound of formula II wherein $Q_1$ and $Q_2$ both represent a nitro group by reaction with a suitable mercaptan, e.g. benzyl mercaptan, in a polar organic solvent such as tetrahydrofuran, to yield a product wherein $Q_1$ is a mercapto grouping and $Q_2$ is nitro, followed by reduction of the nitro group to an amino group by treatment with sodium dihydrogen phosphite ($NaH_2PO_2$) in the presence of palladium/carbon catalyst.

When the desired product is an oxazole, i.e. X is an oxygen atom, Y is the group $CR_4$, and Z is a nitrogen atom, then a suitable starting material of formula II is that wherein $Q_1$ represents a hydroxyl group and $Q_2$ represents an amino group. Cyclization to the desired oxazole ring is suitably effected by reaction with a trialkyl orthoformate such as triethyl orthoformate. Alternatively, when $R_4$ is a thiol or alkylthio group, the cyclization may conveniently be effected by reaction with carbon disulphide in the presence of an alkali, such as sodium hydroxide. The starting material may be prepared from a compound of formula II wherein $Q_1$ represents a hydroxyl group and $Q_2$ represents a nitro group by reduction with sodium dihydrogen phosphite in the presence of palladium/carbon catalyst.

When the desired product is a thiazole, i.e. X is a sulphur atom, Y is the group $CR_4$ and Z is a nitrogen atom, then a suitable starting material of formula II is that wherein $Q_1$ represents an acylthio group, preferably an alkanoylthio group such as acetylthio, and $Q_2$ represents a nitro group. Cyclization to the desired thiazole ring is suitably effected by reaction with stannous chloirde. The starting material may be prepared from a compound of formula II wherein $Q_1$ and $Q_2$ both represent a nitro group by reaction with the appropriate thioalkanoic acid.

When the desired product is a diazole, e.g. an imidazole where X is NH; Z is a nitrogen atom and Y is $CR_4$, then a suitable starting material of formula II is that wherein $Q_1$ and $Q_2$ both represent an amino group. Cyclisation to the desired imidazole ring may be effected by reaction with trifluoroacetic acid/anhydride. Alternatively, a starting material of formula II wherein $Q_1$ represents an acylamino group and $Q_2$ represents a nitro group may be cyclized by reduction with hydrogen in the presence of a palladium/carbon catalyst. In either case, the starting material may be prepared from a compound of formula II wherein $Q_1$ and $Q_2$ both represent a nitro group; in the former case, by reduction with sodium dihydrogen phosphite in the presence of palladium/crbon catalyst; in the latter case by reaction with acetamide.

When the desired product is a triazole, i.e. X is NH and Y and Z are both nitrogen, then a suitable starting material of formula II is that wherein $Q_1$ and $Q_2$ both represent an amino group. Cyclization to the desired triazole is suitably effected by reaction with alkali metal nitrite in the presence of an acid. An alternative starting material of formula II is that wherein $Q_1$ represents an alkylhydrazino group, such as 1-methylhydrazino, and $Q_2$ represents a nitro group. Cyclization to the desired triazole ring is suitably effected by warming with polyphosphoric acid. The starting material may suitably be prepared from a compound of formula II wherein $Q_1$ and $Q_2$ both represent a nitro group by reaction with the appropriate alkylhydrazine.

The compounds of general formula I have been found to show interesting activity as herbicides. Accordingly, the invention further provides a herbicidal composition comprising a compound of formula I as defined above in association with at least one carrier, and a method of making such a composition which comprises bringing a compound of formula I into association with at least one carrier.

The invention also provides the use of such a compound or composition according to the invention as a herbicide. Further, in accordance with the invention there is provided a method of combating undesired plant growth at a locus by treating the locus with a compound or composition according to the invention. Application to the locus may be pre-emergence or post-emergence. The dosage of active ingredient used may, for example, be from 0.05 to 4 kg/ha. A carrier in a composition according to the invention in any material with which the active ingredient is formulated to facilitate application to the locus to be treated which may for example be a plant, seed or soil, or to facilitate storage, transport or handling. A carrier may be a solid or a liquid, including a material which is normally gaseous but which has been compressed to form a liquid, and any of the carriers normally used in formulating herbicidal compositions may be used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

According to another aspect therefore, the invention provides a herbicidal composition, which comprises a compound of the general formula I:—

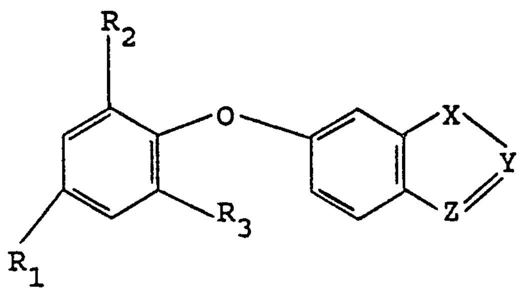

I

wherein

$R_1$ represents a halogen atom or haloalkyl group; $R_2$ and $R_3$, which may be the same or different, each independently represents a hydrogen or halogen atom or a nitro, or haloalkyl group;

one of Y and Z represents a nitrogen atom or an oxide thereof, and the other also represents a nitrogen atom or oxide thereof or alternatively represents the group $CR_4$;

X represents an oxygen or sulphur atom, a carbonyl, thiocarbonyl or sulphonyl group or a group $NR_5$ or $C(R_4)_2$; $R_4$ represents a hydrogen atom or a mercaptan, hydroxyl, alkoxy, alkylthio, alkylsulphonyl, alkyl, amino, alkylureido, alkylcarbonyloxyalkyl, alkoxycarbonylalkylthio, or alkylcarbamoyl group, provided that when two groups $R_4$ are attached to the same carbon atom, then said groups are not both hydroxyl, mercaptan or alkylthio; and

$R_5$ represents a hydrogen atom or a hydroxyl, alkoxy, alkylsulphonyl, alkyl, haloalkyl, amino, alkylureido, alkylcarbonyloxyalkyl or alkylcarbamoyl group, any alkyl or haloalkyl group having up to 6 carbon atoms, provided that when $R_1$ represents a trifluoromethyl group, $R_2$ represents a chlorine atom, $R_3$ represents a hydrogen atom, X represents N—$CH_3$ and Z represents N→O, Y may additionally represent C—CH=$NCH_3$, together with at least two carriers, at least one of which is a surface active agent.

A surface-active agent may be an emulsifying agent, a dispersing agent or a wetting agent; it may be nonionic or ionic. Examples of suitable surface-active agents include the sodium or calcium salts or polyacrylic acids and lignin sulphonic acid; the condensation of fatty acids or aliphatic amines or amides containing at least 12 carbon atoms in the molecule with ethylene oxide and/or propylene oxide; fatty acid esters of glycerol, sorbitol, sucrose or pentaerythritol; condensates of these with ethylene oxide and/or propylene oxide; condensation products of fatty alcohol or alkylphenols, for example *p*-octylphenol or *p*-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 and 75% w of active ingredient and usually contain in addition to solid inert carrier, 3—10% w of a dispersing agent and, where necessary, 0—10% w of stabilizer(s) and/or other additives such as penetrants or stickers. Dusts are usually formulated as a dust concentrate having a similar composition to that of a wettable powder but without a dispersant, and are diluted in the field with further solid carrier to give a composition usually containing ½—10% w of active ingredient. Granules are usually prepared to have a size between 10 and 100 BS mesh (1.676—0.152 mm), and may be manufactured by

agglomeration or impregnation techniques. Generally, granules will contain ½—75% w active ingredient and 0—10% w of additives such as stabilisers, surfactants, slow release modifiers and binding agents. The so-called "dry flowable powders" consist of relatively small granules having a relatively high concentration of active ingredient. Emulsifiable concentrates usually contain, in addition to a solvent and, when necessary, co-solvent, 10—50% w/v active ingredient, 2—20% w/v emulsifiers and 0—20% w/v of other additives such as stabilizers, penetrants and corrosion inhibitors. Suspension concentrates are usually compounded so as to obtain a stable, non-sedimenting flowable product and usually contain 10—75% w active ingredient, 0.5—15% w of dispersing agents, 0.1—10% w of suspending agents such as protective colloids and thixotropic agents, 0—10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick 'mayonnaise'—like consistency.

The composition of the invention may also contain other ingredients, for example other compounds possessing herbicidal, insecticidal or fungicidal properties.

The invention is illustrated in the following examples.

## Example 1

6-(2-chloro-4-trifluoromethylphenoxy)-1,2,3-benzothiadiazole

A) m-nitrophenol (30.6 g) was dissolved in dimethylsulphoxide (100 ml), and potassium hydroxide (14.5 g) added with stirring under nitrogen at 80°C. After 1.5 hours, 3,4-dichlorobenzotrifluoride (43 g) was added dropwise over 30 mins and the temperature increased to 150°C followed by stirring overnight. The bulk of the dimethylsulphoxide was removed, ether/water (1:1 1000 ml) added, the suspension filtered, and the organic layer separated, dried and evaporated to yield an orange oil, which was chromatographically purified. This oil (35 g) was dissolved in ethylene dichloride (70 ml), and conc sulphuric acid added with stirring and cooling. When the reaction mixture was below 2°C, potassium nitrate (11.6 g) was added in portions over 30 minutes. The reaction mixture was then allowed to read ambient temperature, stirred for 3 hours, and then poured into ice water (11) followed by addition of sodium chloride and extraction with ethylene dichloride. The organic layers were separated, dried, solvent removed, chromatographically purified and the product recrystallised to yield, as a solid m.pt. 80—81°C, 4-(2-chloro-4-trifluoromethyl-phenoxy)-1,2-dinitrobenzene.

B) Benzyl mercaptan (1.7 g) was dissolved in dry tetrahydrofuran and sodium hydride (0.5 g) added with stirring under dry nitrogen. The reaction mixture was stirred under reflux for 30 mins, and a solution of the 4-(2-chloro-4-trifluoromethylphenoxy)-1,2-dinitrobenzene (5 g prepared as above) dissolved in dry tetrahydrofuran (25 ml) was added dropwise. Reaction occurred rapidly, and the product was chromatographically purified to give 2-benzylthio-4-(2-chloro-4-trifluoromethylphenoxy) nitrobenzene as a yellow oil.

C) the benzylthio product of B) (2 g) was dissolved in ethanol (30 ml) and 5% palladium on carbon (0.5 g) and 10% aqueous sodium dihydrogen phosphite added portion wise until vigorous effervescence ceased. The reaction mixture was filtered, extracted with a 1:1 mixture of ethylene dichloride/water (400 ml), and the organic layer separated and dried. Chromatographic purification yielded 2-benzylthio-4-(2-chloro-4-trifluoromethylphenoxy) aniline as a brown oil.

D) The aniline product of C) (3 g) was added to an ice-cold solution of 50% aqueous sulphuric acid, and the mixture maintained below 10°C during the addition of a solution of sodium nitrite (0.7 g) in water (10 ml). This mixture was then stirred for 1 hour, allowed to reach ambient temperature, and then extracted with ether. The organic layer was separated, solvent removed and the resulting red oil chromatographically purified and recrystallised to yield the desired product as a crystalline solid, m.pt. 72—73°C.

| Analysis | | | |
|---|---|---|---|
| Calc. for $C_{13}H_6N_2ClF_3OS$: | C 47.2; | H 1.8; | N 8.5% |
| Found | C 47.1; | H 1.7; | N 8.2% |

## Example 2

Following synthetic procedures similar to those of Example 1, there was prepared 6-(2-nitro-4-trifluoro-methylphenoxy)-1,2,3-benzothiadiazole as a solid, m.pt. 97—99°C.

## Example 3

6-(2-chloro-4-trifluoromethylphenoxy)-1,3-benzoxazole

A) 2-hydroxy-4-(2-chloro-4-trifluoromethylphenoxy)nitrobenzene (3.33 g) was dissolved in hot ethanol (250 ml) and 5% palladium on carbon (0.25 g) added under nitrogen followed by dropwise addition of sodium dihydrogen phosphite (60 ml) over 2 hours. The reaction mixture was refluxed for an hour, allowed to cool, filtered and the residue extracted with methylene chloride/water. The organic layer was water washed, dried and solvent removed to yield the aniline derivative as a brown oil.

B) The aniline of B) (3.0 g) was refluxed with ethyl orthoformate (200 ml) for 5 hours. Solvent was removed and the residue oil chormatographically purified to yield the desired product as a pale yellow oil whose structure was confirmed by n.m.r. [?Boiling Point?]

Analysis

| | | | |
|---|---|---|---|
| Calc. for $C_{14}H_7ClF_3NO_2$: | C 53.6; | H 2.2; | N 4.5% |
| Found | C 53.7; | H 2.2; | N 4.3% |

Example 4

2-mercapto-6-(2-chloro-4-trifluoromethylphenoxy)-1,3-benzoxazole

2-hydroxy-4-(2-chloro-4-trifluoromethylphenoxy) aniline (prepared as in Example 3(A); 6g) was dissolved in 2N sodium hydroxide (60 ml) and carbon disulphide (3 g) added at 0°C. The reaction mixture was allowed to reach ambient temperature overnight, neutralised with dilute acetic acid and water/ether (1:1; 500 ml) added. The organic layer was separated, washed, dried and chromatographically purified to yield the desired product as a solid, m.pt. 165°C.

Analysis

| | | | |
|---|---|---|---|
| Calc. for $C_{15}H_9NClF_3O_2S$: | C 48.6; | H 2.0; | N 4.1% |
| Found | C 48.6; | H 2.0; | N 4.1% |

Example 5

2-methylthio-6-(2-chloro-4-trifluoromethylphenoxy)-1,3-benzoxazole

The mercaptan of Example 4 (4 g) was dissolved in 10% aqueous sodium hydroxide (4 fold excess) and dimethylsulphate (3 g) added dropwise at ambient temperature, giving an almost immediate precipitate of the alkylated product. Water/ether (1:1; 400 ml) was added, and the organic layer separated, washed, dried and solvent removed to yield a colourless oil. Recrystallisation gave the desired product as a solid, m.pt. 75°C.

Analysis

| | | | |
|---|---|---|---|
| Calc. for $C_{15}H_9NClF_3O_2S$: | C 50.0; | H 2.5; | N 3.9% |
| Found | C 50.0; | H 2.5; | N 3.9% |

Example 6

2-methylsulphonyl-6-(2-chloro-4-trifluoromethylphenoxy)-1,3-benzoxazole

The methylthio compound of Example 5 (4.4 g) was dissolved in acetic acid (25 ml) and a solution of potassium permanganate (5.5 g) in water (90 ml) added dropwise with cooling and stirring, whilst maintaining the temperature below 30°C. The reaction mixture was stirred for 2 hours, then poured into ice-water and sulphur dioxide bubbled through until the solution became clear. Ethylene dichloride (300 ml) was added, and the organic layer separated, washed and dried to yield a colourless oil. Recrystallisation gave the desired product as a solid, m.pt. 110°C.

Analysis

| | | | |
|---|---|---|---|
| Calc. for $C_{15}H_9NClF_3O_4S$: | C 46.0; | H 2.3; | N 3.6% |
| Found | C 46.1; | H 2.3; | N 3.5% |

Example 7

2-methylsulphonyl-6-(2-chloro-4-trifluoromethylphenoxy)-1,3-benzoxazole, N oxide

The methylsulphonyl compound of Example 6 (1 g) and m-chloro perbenzoic acid (0.5 g) dissolved in ethylene dichloride were stirred at 20°C for 2 days. Chromatographic purification and recrystallisation yielded the desired product as a solid, m.pt 160°C.

Analysis

| | | | |
|---|---|---|---|
| Calc. for $C_{15}H_7NClF_3O_5S$: | C 44.1; | H 2.2; | N 3.4% |
| Found | C 43.9; | H 2.2; | N 3.4% |

Examples 8—10

Following procedures similar to those described in Examples 3 or 4, further benzoxazoles of the invention were prepared, whose physical characteristics and chemical analyses are given in Table 1 below. In that Table, the compounds are identified by reference to the identity of the $R_4$ substituent in the following formula:—

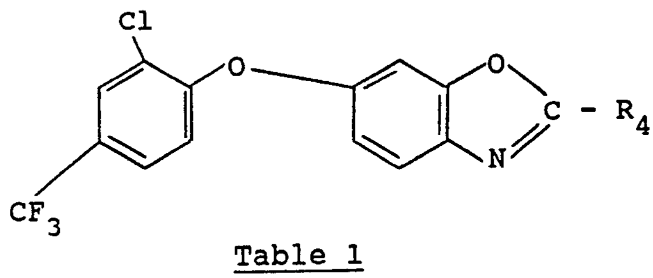

Table 1

| | | | Analysis | | | | | |
|---|---|---|---|---|---|---|---|---|
| Ex. | | M.Pt. | Calc. | | | Found | | |
| No. | $R_4$ | °C | C | H | N | C | H | N |
| 8 | $CH_3$ | oil | 55.0 | 2.7 | 4.3 | 55.3 | 3.2 | 4.1 |
| 9 | $OCH_3$ | oil | 52.4 | 2.6 | 4.1 | 52.4 | 2.6 | 4.1 |
| 10 | $SC(CH_3)HCOOC_2H_5$ | oil | 51.2 | 3.4 | 3.1 | 49.5 | 3.5 | 2.9 |

Example 11

2-methyl-6-(2-chloro-4-trifluoromethylphenoxy-1,3-benzothiazole

A) Sodium hydride (0.65 g) was added to a solution of thioacetic acid (2.1 g) in tetrahydrofuran (50 ml). 4-(2-chloro-4-trifluoromethylphenoxy)-1,2-dinitrobenzene (prepared as in Example 1A; 10 g) in tetrahydrofuran (50 ml) was added dropwise, and the reaction mixture stirred at ambient temperature for 30 mins. The reaction mixture was poured into ether/water (1:1; 500 ml), and the organic layer separated, dried and solvent removed to yield a brown oil. This was identified as 2-acetylthio-4-(2-chloro-4-trifluoromethylphenoxy) nitrobenzene.

B) The acetylthio compound of A) (7.0 g) was dissolved in ethanol (50 ml) and stannous chloride (20 g) added. The reaction mixture was heated to 70°C for 30 mins., and after cooling was poured into ice water and 10% aqueous sodium hydroxide added to adjust the pH to 8. After filtration, the solution was extracted with ethylene dichloride, the organic phase separated, dried and evaporated to yield a brown oil. Chromatographic purification yielded the desired product as an orange oil whose structure was confirmed by n.m.r.

Analysis

| | | | |
|---|---|---|---|
| Calc. for $C_{15}H_9ClF_3NOS$: | C 52.4; | H 2.6; | N 4.1% |
| Found | C 48.7; | H 2.5; | N 3.9% |

Example 12

5-(2-chloro-4-trifluoromethylphenoxy)1,2,3-benzotriazole

2-amino-4-(2-chloro-4-trifluoromethylphenoxy)aniline (prepared by reduction of the corresponding dinitro compound, as described in the following example; 6g) was dissolved in a mixture of glacial acetic acid (4 ml) and water (5 ml), cooled to 15°C, and a solution of sodium nitrite (1.25 g) in water (6 ml) added with stirring. The reaction mixture was allowed to cool, and water/ethylene dichloride (1:1; 200 ml) added. The organic layer was separated, dried, chromatographically purified and recrystallised to yield the desired product as a solid, m.pt. 105°C.

Analysis

| | | | |
|---|---|---|---|
| Calc. for $C_{13}H_7N_3ClF_3O$: | C 49.8; | H 2.2; | N 13.4% |
| Found | C 49.6; | H 2.1; | N 13.3% |

Example 13

3-(N-methylcarbamoyl)-5-(2-chloro-4-trifluoromethylphenoxy)-1,2,3-benzotriazole

The benzotriazole product of Example 12 (2 g) was dissolved in warm toluene (50 ml) with methylisocyanate (0.7 g) and the reaction mixture refluxed under nitrogen with stirring for 1 hour. Chromatographic separation and recrystallisation yield the desired, title product as a solid, m.pt. 122°C.

Analysis

| | | | |
|---|---|---|---|
| Calc. for $C_{15}H_{10}N_4ClF_3O_2$: | C 48.6; | H 2.7; | N 15.1% |
| Found | C 48.5; | H 2.6; | N 15.0% |

Example 14

1,2-dimethyl-5-(2-chloro-4-trifluoromethylphenoxy)-1,3-benzimidazole, 1 N-oxide

4-(2-chloro-4-trifluoromethylphenoxy)-1,2-dinitrobenzene (prepared as in Example 1A) was reacted

with acetamide in the presence of sodium hydride in tetrahydrofuran. The resulting acetylamino derivative (10 g) was dissolved in dimethylformamide (50 ml) and potassim tert-butoxide (3.0 g) added with stirring under nitrogen. After ½ hour methyl iodide (6.0 g) in dimethylformamide (50 ml) was added dropwise, the temperature raised to 50°C and the reaction mixture stirred for 4 hours. It was then poured into ethyl/water (1:1, 50 ml), the ether layer separated, dried and chromatographically purified to yield a yellow oil of the N-methyl derivative. This compound (8.0 g) was then mixed with ethanol (48 ml), concentrated hydrochloric acid (3.2 ml) and 5% palladium on carbon (0.8 g) and hydrogenated with hydrogen gas in a Parr hydrogenator until no more hydrogen was absorbed. The reaction mixture was neutralised with dilute sodium hydroxide and extracted with ethylene dichloride. The solvent was evaporated off and the solid residue recrystallised to yield the desired product, m.pt. 143—144°C.

Analysis

| | | | |
|---|---|---|---|
| Calc. for $C_{16}H_{12}ClF_3N_2O_2$: | C 53.9; | H 3.4; | N 7.8% |
| Found | C 52.2; | H 3.7; | N 7.4% |

Examples 15—22

Following the procedures similar to those described in Example 14, further benzimidazoles of the invention were prepared, whose physical characteristics and chemical analyses are given in Table 2 below. In this Table, the compounds are identified by reference to the identity of the $R_4$ substituent in the following formula:—

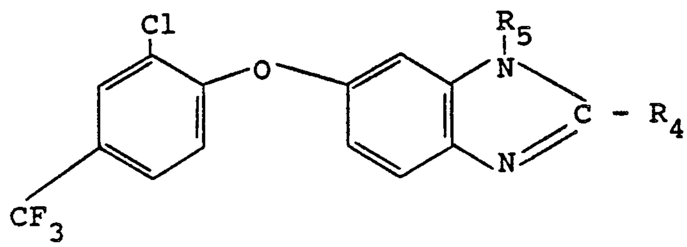

Table 2

| Ex. No. | $R_4$ | $R_5$ | M.Pt. °C | Analysis Calc. C | Calc. H | Calc. N | Found C | Found H | Found N |
|---|---|---|---|---|---|---|---|---|---|
| 15 | H | H | 166 | 53.7 | 2.6 | 9.0 | 53.7 | 2.6 | 8.8 |
| 16 | $CH_3$ | H | 185 | 55.1 | 3.1 | 8.6 | 55.3 | 3.2 | 8.5 |
| 17 | $SCH_3$ | H | 148-50 | 50.2 | 2.8 | 7.8 | 50.4 | 2.8 | 7.7 |
| 18 | $SO_2CH_3$ | H | 125-6 | 46.1 | 2.6 | 7.2 | 46.0 | 2.2 | 6.8 |
| 19 | $NH_2$ | H | 72-3 | 51.2 | 2.7 | 12.8 | 51.3 | 3.2 | 12.2 |
| 20 | $NHCONHCH_3$ | H | 188-9 | 49.9 | 3.1 | 14.6 | 49.6 | 3.4 | 14.4 |
| 21 | $CH_2OCOCH_3$ (N-Oxide) | $CH_3$ | 114-6 | 54.2 | 3.5 | 7.0 | 54.2 | 3.5 | 7.0 |
| 22 | $CH{=}NCH_3$ (N-oxide) | $CH_3$ | 240 | 49.8 | 2.9 | 10.9 | 49.8 | 2.9 | 10.6 |

Example 23

3-Methyl-5-(2-chloro-4-trifluoromethylphenoxy)-1,2,3-benzotriazole, I oxide

A) (2'-chloro-4'-trifluoromethylphenoxy)-3,4-dinitro benzene (5 g) was dissolved in dioxan (50 ml) and methylhydrazine (0.8 g) added dropwise with stirring under nitrogen, with immediate reaction. The reaction mixture was chromatographically separated and recrystallised to yield the 3-(alpha methyl)-hydrazine derivative, m.pt 100°C.

B) The hydrazine product of A (1.5 g) was dissolved in polyphosphoric acid (10 ml) and stirred under nitrogen. The reaction mixture was heated at 50°C for ½ hr., then poured onto ice and extracted into chloroform. Chromatographic separted yielded the desired product as a colourless solid, m.pt. 170°C.

Analysis

| | | | |
|---|---|---|---|
| Calc. for $C_{14}H_9N_3ClF_3O_2$: | C 48.9; | H 2.6; | N 12.2% |
| Found | C 48.8; | H 2.3; | N 11.9% |

Example 24

Herbicidal Activity

To evaluate their herbicidal activity, compounds according to the invention were tested using as representative range of plants: maize, *Zea mays* (Mz); rice, *Oryza sativa* (R); barnyard grass, *Echinochloa crusgalli* (BG); oat, *Avena sativa* (O); linseed, *Linum usitatissimum* (L); mustard, *Sinapsis alba* (M); sugar beet, *Beta vulgaris* (SB) and soya bean, *Glycine max* (S).

The tests fall into two categories, pre-emergence and post-emergence. The pre-emergence tests involved spraying a liquid formulation of the compound onto the soil in which the seeds of the plant species mentioned above had recently been sown. The post-emergence tests involved two types of test, viz., soil drench and foliar spray tests. In the soil drench tests the soil in which the seedling plants of the above species were growing was drenched with a liquid formulation containing a compound of the invention, and in the folair spray tests the seedling plants were sprayed with such a formulation.

The soil used in the tests was a prepared horticultural loam.

The formulations used in the tests were prepared from solutions of the test compounds in acetone containing 0.4% by weight of an alkylphenol/ethylene oxide condensate available under the trade mark TRITON X—155. These acetone solutions were diluted with water and the resulting formulations applied at dosage levels corresponding to 5 kg or 1 kg of active material per hectare in a volume equivalent to 600 litres per hectare in the soil spray and foliar spray test, and at a dosage level equivalent to 10 kilograms of active material per hectare in a volume equivalent to approximately 3,000 litres per hectare in the soil drench tests.

In the pre-emergence tests untreated sown soil and in the post-emergence tests untreated soil bearing seedling plants were used as controls.

The herbicidal effects of the test compounds were assessed visually twelve days after spraying the foliage and the soil, and thirteen days after drenching the soil and were recorded on a 0—9 scale. A rating 0 indicates growth as untreated control, a rating 9 indicates death. An increase of 1 unit on the linear scale approximates to a 10% increase in the level of effect.

The results of the tests are set out in Table 3 below, in which the compounds are identified by reference to the preceding examples.

Table 3

| Compound of Ex. No. | Soil drench 10/kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 1 | 5 | 5 | 4 | 7 | 6 | 2 | 7 | 2 | 5 | 8 | 6 | 9 | 9 | 9 | 9 | 9 | 9 | 7 | 7 | 9 | 7 | 6 | 6 | 9 | 0 |
| | | | | | | | | | 1 | 7 | 4 | 9 | 8 | 9 | 8 | 9 | 8 | 6 | 6 | 9 | 6 | 5 | 4 | 8 | 0 |
| 2 | 0 | 0 | 0 | 0 | 0 | 0 | 0 | 0 | 5 | 7 | 5 | 8 | 5 | 6 | 9 | 9 | 7 | 0 | 3 | 5 | 0 | 0 | 4 | 8 | 2 |
| | | | | | | | | | 1 | 5 | 4 | 5 | 4 | 5 | 8 | 7 | 4 | 0 | 2 | 4 | 0 | 0 | 2 | 6 | 0 |
| 3 | 0 | 0 | 3 | 2 | 3 | 3 | 5 | 3 | 5 | 7 | 4 | 7 | 3 | 7 | 7 | 8 | 6 | 0 | 0 | 6 | 2 | 3 | 3 | 5 | 0 |
| | | | | | | | | | 1 | 6 | 3 | 6 | 2 | 6 | 2 | 6 | 6 | 0 | 0 | 2 | 1 | 2 | 2 | 2 | 0 |
| 8 | 0 | 0 | 0 | 0 | 0 | 0 | 0 | 0 | 5 | 5 | 4 | 4 | 4 | 7 | 3 | 8 | 5 | 2 | 0 | 2 | 0 | 0 | 0 | 0 | 0 |
| | | | | | | | | | 1 | 5 | 3 | 3 | 2 | 4 | 3 | 6 | 4 | 1 | 0 | 1 | 0 | 0 | 0 | 0 | 0 |
| 10 | 3 | 0 | 0 | 0 | 5 | 5 | 7 | 4 | 5 | 5 | 5 | 7 | 6 | 9 | 8 | 9 | 7 | 0 | 0 | 4 | 3 | 6 | 5 | 9 | 0 |
| | | | | | | | | | 1 | 4 | 4 | 5 | 6 | 7 | 7 | 8 | 5 | 0 | 0 | 4 | 1 | 3 | 2 | 6 | 0 |

Table 3 (contiued)

| Compound of Ex. No. | Soil drench 10/kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | kg/ha | Mz | R | BG | O | L | M | SB | S | Mz | R | BG | O | L | M | SB | S |
| 11 | 0 | 0 | 0 | 0 | 0 | 0 | 0 | 0 | 5 | 6 | 5 | 6 | 6 | 8 | 3 | 8 | 6 | 0 | 0 | 0 | 0 | 0 | 0 | 0 | 0 |
| | | | | | | | | | 1 | 4 | 3 | 4 | 4 | 7 | 5 | 5 | 4 | 0 | 0 | 0 | 0 | 0 | 0 | 0 | 0 |
| 12 | 0 | 0 | 0 | 0 | 0 | 0 | 0 | 0 | 5 | 4 | 0 | 4 | 4 | 5 | 4 | 6 | 5 | 0 | 0 | 0 | 0 | 0 | 0 | 0 | 0 |
| | | | | | | | | | 1 | 2 | 0 | 2 | 1 | 4 | 2 | 2 | 2 | 0 | 0 | 0 | 0 | 0 | 0 | 0 | 0 |
| 14 | 0 | 0 | 0 | 0 | 0 | 0 | 0 | 0 | 5 | 4 | 2 | 4 | 3 | 7 | 5 | 5 | 4 | 0 | 0 | 0 | 3 | 2 | 3 | 8 | 1 |
| | | | | | | | | | 1 | 2 | 2 | 3 | 2 | 6 | 4 | 5 | 3 | 0 | 0 | 0 | 3 | 1 | 3 | 6 | 0 |
| 21 | 0 | 0 | 0 | 0 | 0 | 0 | 0 | 0 | 5 | 2 | 3 | 3 | 2 | 8 | 7 | 4 | 5 | 0 | 0 | 0 | 0 | 0 | 0 | 0 | 0 |
| | | | | | | | | | 1 | 1 | 2 | 3 | 1 | 6 | 5 | 3 | 4 | 0 | 0 | 0 | 0 | 0 | 0 | 0 | 0 |
| 23 | 8 | 8 | 8 | 8 | 9 | 9 | 8 | 8 | 5 | 6 | 5 | 9 | 8 | 9 | 9 | 9 | 7 | 8 | 8 | 9 | 7 | 9 | 9 | 9 | 6 |
| | | | | | | | | | 1 | 6 | 3 | 8 | 6 | 9 | 8 | 9 | 7 | 7 | 5 | 8 | 6 | 7 | 8 | 9 | 2 |

**Claims for the Contracting States: BE CH DE FR GB IT LI LU NL SE**

1. Diphenyl ethers having the general formula I:—

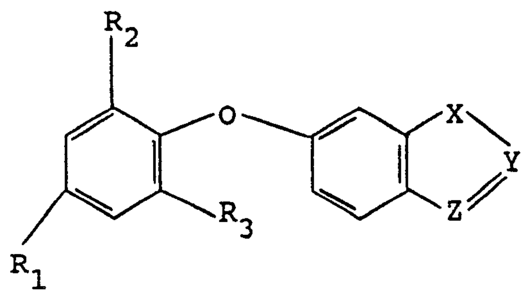

I

wherein

$R^1$ represents a haloalkyl group;

$R_2$ and $R_3$, which may be the same or different, each independently represents a hydrogen or halogen atom or a nitro, or haloalkyl group;

one of Y and Z represents a nitrogen atom or an oxide thereof, and the other also represents a nitrogen atom or oxide thereof or alternatively represents the group $CR_4$;

X represents an oxygen or sulphur atom, a carbonyl, thiocarbonyl or sulphonyl group or a group $NR_5$ or $C(R_4)_2$;

$R_4$ represents a hydrogen atom or a mercaptan, hydroxyl, alkoxy, alkylthio, alkylsulphonyl, alkyl, amino, alkylureido, alkylcarbonyloxyalkyl, alkoxycarbonylalkylthio, or alkylcarbamoyl group, provided that when two groups $R_4$ are attached to the same carbon atom, then said groups are not both hydroxyl, mercaptan or alkylthio; and

$R_5$ represents a hydrogen atom or a hydroxyl alkoxy, alkylsulphonyl, alkyl, haloalkyl, amino, alkylureido, alkylcarbonyloxyalkyl or alkylcarbamoyl group, any alkyl or haloalkyl group having up to 6 carbon atoms, provided that when $R_1$ represents a trifluoromethyl group, $R_2$ represents a chlorine atom, $R_3$ represents a hydrogen atom, X represents $N—CH_3$ and Z represents $N \rightarrow O$, Y may additionally represent $C—CH{=}NCH_3$.

2. Compounds as claimed in claim 1 wherein $R_1$ is a trifluoromethyl group; $R_2$ is a chlorine atom or a nitro group, and $R_3$ is a hydrogen atom.

3. Compounds as claimed in claim 1 or 2 wherein Z represents a nitrogen atom or an oxide thereof, and X represents an oxygen or sulphur atom or the group $NR_5$ in which $R_5$ represents a hydrogen atom or an alkyl or alkylcarbamoyl group.

4. Compounds as claimed in claim 1, 2 or 3 wherein Y represents a nitrogen atom or oxide thereof, or the group $CR_4$ in which $R_4$ represents a hydrogen atom or an amino or mercaptan group or an alkylthio, alkylsulphonyl, alkoxy, alkyl, alkoxycarbonylalkylthio, alkylcarbonyloxyalkyl, or alkylureido group in which each alkyl moiety contains 1 to 4 carbon atoms.

5. Process for the preparation of a compound of the general formula I in which $R_1$, $R_2$ and $R_3$ are as defined in claim 1, X is sulphur and Y and Z are both nitrogen atoms, which comprises reacting a compound of formula II:

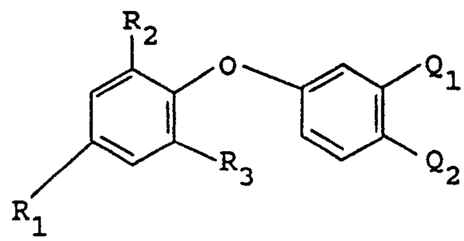

II

wherein $Q_1$ represents a protected mercapto group and $Q_2$ represents an amino group, with an aqueous solution of an alkali metal nitrite in the presence of an acid at a temperature not exceeding 10°C.

6. Process for the preparation of a compound of the general formula I in which $R_1$, $R_2$ and $R_3$ are as defined in claim 1, X is oxygen, Y is the group CH and Z is a nitrogen atom, which comprises reacting a compound of formula II wherein $Q_1$ represents a hydroxyl group and $Q_2$ represents an amino group, with an orthoformate.

7. Process for the preparation of a compound of the general formula I in which $R_1$, $R_2$ and $R_3$ are as defined in claim 1, X is a sulphur arom, Y is the group $CR_4$ and Z is a nitrogen atom, which comprises reacting a compound of formula II, wherein $Q_1$ represents an acylthio group and $Q_2$ represents a nitro group, with stannous chloride.

8. Process for the preparation of a compound of the general formula I in which $R_1$, $R_2$ and $R_3$ are as defined in claim 1, X is NH and Y and Z are both nitrogen, which comprises reacting a compound of formula II wherein $Q_1$ and $Q_2$ both represent an amino group with an alkali metal nitride in the presence of an acid.

9. Herbicidal composition, which comprises a compound as claimed in any of claims 1 to 4, together with a carrier.

10. Herbicidal composition, which comprises a compound of the general formula I:—

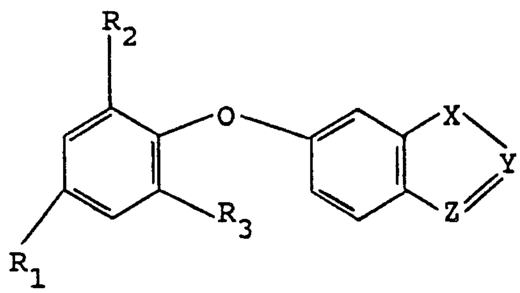

I

wherein

$R_1$ represents a halogen atom or haloalkyl group;

$R_2$ and $R_3$, which may be the same or different, each independently represents a hydrogen or halogen atom or a nitro, or haloalkyl group;

one of Y and Z represents a nitrogen atom or an oxide thereof, and the other also represents a nitrogen atom or oxide thereof or alternatively represents the group $CR_4$;

X represents an oxygen or sulphur atom, a carbonyl, thiocarbonyl or sulphonyl group or a group $NR_5$ or $C(R_4)_2$;

$R_4$ represents a hydrogen atom or a mercaptan, hydroxyl, alkoxy, alkylthio, alkylsulphonyl, alkyl, amino, alkylureido, alkylcarbonyloxyalkyl, alkoxycarbonylalkylthio, or alkylcarbamoyl group, provided that when two groups $R_4$ are attached to the same carbon atom, then said groups are not both hydroxyl, mercaptan or alkylthio; and

$R_5$ represents a hydrogen atom or a hydroxyl alkoxy, alkylsulphonyl, alkyl, haloalkyl, amino, alkylureido, alkylcarbonyloxyalkyl or alkylcarbamoyl group, any alkyl or haloalkyl group having up to 6 carbon atoms, provided that when $R_1$ represents a trifluoromethyl group, $R_2$ represents a chlorine atom, $R_3$ represents a hydrogen atom, X represents $N—CH_3$ and Z represents $N{\rightarrow}O$, Y may additionally represent $C—CH{=}NCH_3$, together with at least two carriers, at least one of which is a surface active agent.

11. Method of combating undesired plant growth at a locus, which comprises treating the locus with a compound as claimed in any one of claims 1 to 4, or a composition as claimed in claim 9 or 10.

12. The use of a compound as claimed in any one of claims 1 to 4, as a herbicide.

**Claims for the Contracting State: AT**

1. Herbicidal composition, which comprises a compound of the general formula I:—

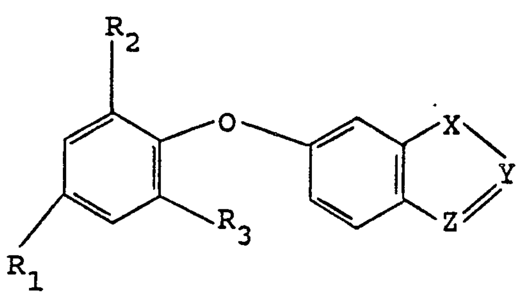

I

wherein

$R^1$ represents a halogen atom or haloalkyl group;

$R_2$ and $R_3$, which may be the same or different, each independently represents a hydrogen or halogen atom or a nitro, or haloalkyl group;

one of Y and Z represents a nitrogen atom or an oxide thereof, and the other also represents a nitrogen atom or oxide thereof or alternatively represents the group $CR_4$;

X represents an oxygen or sulphur atom, a carbonyl, thiocarbonyl or sulphonyl group or a group $NR_5$ or $C(R_4)_2$;

$R_4$ represents a hydrogen atom or a mercaptan, hydroxyl, alkoxy, alkylthio, alkylsulphonyl, alkyl, amino, alkylureido, alkylcarbonyloxyalkyl, alkoxycarbonylalkylthio, or alkylcarbamoyl group, provided that when two groups $R_4$ are attached to the same carbon atom, then said groups are not both hydroxyl, mercaptan or alkylthio; and

$R_5$ represents a hydrogen atom or a hydroxyl alkoxy, alkylsulphonyl, alkyl, haloalkyl, amino, alkylureido, alkylcarbonyloxyalkyl or alkylcarbamoyl group, any alkyl or haloalkyl group having up to 6 carbon atoms, provided that when $R_1$ represents a trifluoromethyl group, $R_2$ represents a chlorine atom, $R_3$ represents a hydrogen atom, X represents $N—CH_3$ and Z represents $N{\rightarrow}O$, Y may additionally represent

C—CH=$NCH_3$, together with at least two carriers, at least one of which is a surface active agent.

2. A composition as claimed in claim 1 wherein $R_1$ is a trifluoromethyl group; $R_2$ is a chlorine atom or a nitro group, and $R_3$ is a hydrogen atom.

3. A composition as claimed in claim 1 or 2 wherein Z represents a nitrogen atom or an oxide thereof, and X represents an oxygen or sulphur atom or the group $NR_5$ in which $R_5$ represents a hydrogen atom or an alkyl or alkylcarbamoyl group.

4. A composition as claimed in claim 1, 2 or 3 wherein Y represents a nitrogen atom or oxide thereof, or the group $CR_4$ in which $R_4$ represents a hydrogen atom or an amino or mercaptan group or an alkylthio, alkylsulphonyl, alkoxy, alkyl, alkoxycarbonylalkylthio, alkylcarbonyloxyalkyl, or alkylureido group in which each alkyl moiety contains 1 to 4 carbon atoms.

5. Process for the preparation of a compound of the general formula I defined in claim 1 in which $R_1$ represents a haloalkyl group, $R_2$ and $R_3$ are as defined in claim 1, X is sulphur and Y and Z are both nitrogen atoms, which comprises reacting a compound of formula II:

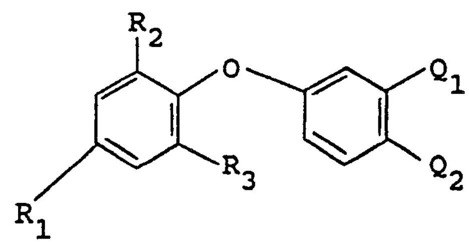

II

wherein $Q_1$ represents a protected mercapto group and $Q_2$ represents an amino group, with an aqueous solution of an alkali metal nitrite in the presence of an acid at a temperature not exceeding 10°C.

6. Process for the preparation of a compound of the general formula I defined in claim 1 in which $R_1$ represents a haloalkyl group, $R_2$ and $R_3$ are as defined in claim 1, X is oxygen, Y is the group CH and Z is a nitrogen atom, which comprises reacting a compound of formula II wherein $Q_1$ represents a hydroxyl group and $Q_2$ represents an amino group, with an orthoformate.

7. Process for the preparation of a compound of the general formula I defined in claim 1 in which $R_1$ represents a haloalkyl group, $R_2$ and $R_3$ are as defined in claim 1, X is a sulphur atom, Y is the group $CR_4$ and Z is a nitrogen atom, which comprises reacting a compound of formula II wherein $Q_1$ represents an acylthio group and $Q_2$ represents a nitro group, with stannous chloride.

8. Process for the preparation of a compound of the general formula I defined in claim 1 in which $R_1$ represents a haloalkyl group, $R_2$ and $R_3$ are as defined in claim 1, X is NH and Y and Z are both nitrogen, which comprises reacting a compound of formula II wherein $Q_1$ and $Q_2$ both represent an amino group with an alkali metal nitride in the presence of an acid.

9. Method of combating undesired plant growth at a locus, which comprises treating the locus with a compound as defined in any one of claims 1 to 4.

10. The use of a compound as defined in any one of claims 1 to 4, as a herbicide.

**Patentansprüche für die Vertragsstaaten: BE CH DE FR GB IT LI LU NL SE**

1. Diphenylether der allgemeinen Formel I:

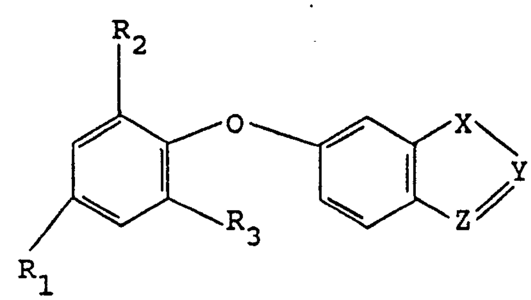

I

worin

$R_1$ eine Halogenalkylgruppe bedeutet;

$R_2$ und $R_3$, die gleich oder verschieden sein können, jeweils unabhängig voneinander ein Wasserstoff- oder Halogenatom oder ene Nitro- oder Halogenalkylgruppe bedeuten;

einer der Reste von Y und Z ein Stickstoffatom oder ein Oxid hievon darstellt und der andere Rest ein Stickstoffatom oder ein Oxid hievon oder die Gruppe C—$R_4$ bedeutet;

X ein Sauerstoff- oder Schwefelatom, eine Carbonyl-, Thiocarbonyl- oder Sulfonylgruppe oder eine Gruppe N—$R_5$ oder $C(R_4)_2$ darstellt;

$R_4$ ein Wasserstoffatom oder eine Mercaptan-, Hydroxyl-, Alkoxy-, Alkylthio-, Alkylsulfonyl-, Alkyl-, Amino-, Alkylureido-, Alkylcarbonyloxyalkyl, Alkoxycarbonylalkylthio- oder Alkylcarbamoylgruppe

darstellt, mit der Maßgabe, daß dann, wenn zwei Gruppen $R_4$ an das gleiche Kohlenstoffatom gebunden sind, diese Gruppen nicht beide Hydroxyl, Mercapto oder Alkylthio bedeuten; und

$R_5$ ein Wasserstoffatom oder eine Hydroxyl-, Alkoxy-, Alkylsulfonyl-, Alkyl-, Halogenalkyl-, Amino-, Alkylureido-, Alkylcarbonyloxyalkyl- oder Alkylcarbamoylgruppe darstellt, wobei eine Alkyl- oder Halogenalkylgruppe bis zu 6 Kohlenstoffatome aufweist mit der Maßgabe, daß dann, wenn $R_1$ eine Trifluormethylgruppe darstellt, $R_2$ ein Chloratom bedeutet, $R_3$ ein Wasserstoffatom darstellt, X für N—$CH_3$ und Z für N → O stehen, Y zusätzlich C—CH=$NCH_3$ bedeuten kann.

2. Verbindungen nach Anspruch 1, worin $R_1$ eine Trifluormethylgruppe bedeutet; $R_2$ ein Chloratom oder eine Nitrogruppe darstellt und $R_3$ ein Wasserstoffatom bedeutet.

3. Verbindung nach Anspruch 1 oder 2, worin Z ein Stickstoffatom oder ein Oxid hievon bedeutet und X für ein Sauerstoff oder Schwefelatom oder eine Gruppe $NR_5$ steht, worin $R_5$ ein Wasserstoffatom oder eine Alkyl- oder Alkylcarbamoylgruppe darstellt.

4. Verbindung nach Anspruch 1, 2 oder 3, worin Y ein Stickstoffatom oder ein Oxid hievon bedeutet oder für die Gruppe $CR_4$ steht, worin $R_4$ ein Wasserstoffatom oder eine Amino- oder Mercaptangruppe oder eine Alkylthio-, Alkylsulfonyl-, Alkoxy-, Alkyl-, Alkoxycarbonylalkylthio-, Alkylcarbonyloxyalkyl- oder Alkylureidogruppe bedeutet, worin jeder Alkylrest 1 bis 4 Kohlenstoffatome enthält.

5. Verfahren zur Herstellung einer Verbindung der allgemeinen Formel I, worin $R_1$, $R_2$ und $R_3$ wie in Anspruch 1 definiert sind, X für Schwefel steht und Y und Z beide Stickstoffatome bedeuten, welches ein Umsetzen einer Verbindung der Formel II:

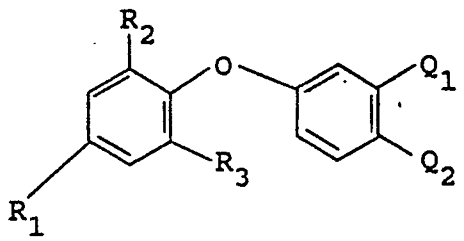

II

worin $Q_1$ eine geschützte Mercaptogruppe darstellt und $Q_2$ eine Aminogruppe bedeutet, mit einer wäßringen Lösung eines Alkalimetallnitrits in Anwesenheit einer Säure bei einer 10°C nicht überschreitenden Temperatur umfaßt.

6. Verfahren zur Herstellung einer Verbindung der allgemeinen Formel I, worin $R_1$, $R_2$ und $R_3$ wie in Anspruch 1 definiert sind, X für Sauerstoff steht und Y die Gruppe CH bedeutet und Z ein Stickstoffatom darstellt, welches Verfahren ein Umsetzen einer Verbindung der Formel II, worin $Q_1$ eine Hydroxylgruppe darstellt und $Q_2$ eine Aminogruppe bedeutet, mit einem Orthoformiat umfaßt.

7. Verfahren zur Herstellung einer Verbindung der allgemeinen Formel I, worin $R_1$, $R_2$ und $R_3$ wie in Anspruch 1 definiert sind, X ein Schewefelatom bedeutet, Y für die Gruppe $CR_4$ steht und Z ein Stickstoffatom darstellt, welches Verfahren ein Umsetzen einer Verbindung der Formel II, worin $Q_1$ eine Acylthiogruppe darstellt und $Q_2$ eine Nitrogruppe beduetet, mit Zinn(II)-chlorid umfaßt.

8. Verfahren zur Herstellung einer Verbindung der allgemeinen Formel I, worin $R_1$, $R_2$ und $R_3$ wie in Anspruch 1 definiert sind, X für NH steht und Y und Z beide Stickstoff bedeuten, welches Verfahren ein Umsetzen einer Verbindung der Formel II, worin $Q_1$ und $Q_2$ beide eine Aminogruppe bedeuten mit einem Alkalimetallnitrit in Anwesenheit einer Säuer umfaßt.

9. Herbicide Zusammensetzung, welche eine Verbindung, wie in einem der Ansprüche 1 bis 4 beansprucht, zusammen mit einem Träger umfaßt.

10. Herbicide Zusammensetzung, welche eine Verbindung der allgemeinen Formel I:

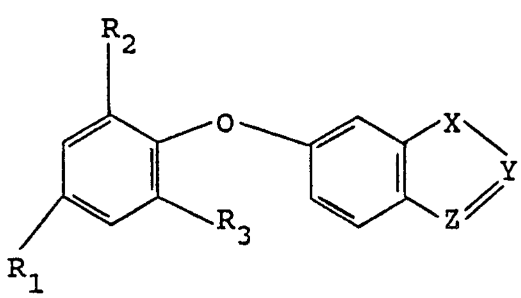

I

umfaßt, worin

$R_1$ ein Halogenatom oder eine Halogenalkylgruppe darstellt;

$R_2$ und $R_3$ die gleich oder verschieden sein können, jeweils unabhängig voneinander ein Wasserstoff- oder Halogenatom oder eine Nitro- oder Halogenalkylgruppe bedeuten;

einer der Reste von Y und Z ein Stickstoffatom oder ein oxid hievon darstellt und der andere Rest ebenfalls ein Stickstoffatom oder Oxid hievon bedeutet oder in alternativer Weise die Gruppe $CR_4$ bedeutet;

X ein Sauerstoff- oder Schwefelatom, eine Carbonyl-, Thiocarbonyl- oder Sulfonylgruppe oder eine Gruppe $NR_5$ oder $C(R_4)_2$ bedeutet; $R_4$ ein Wasserstoffatom oder eine Mercaptan-, Hydroxyl-, Alkoxy-, Alkylthio-, Alkylsulfonyl-, Alkyl-, Amino-, Alkylureido-, Alkylcarbonyloxyalkyl-, Alkoxycarbonylalkylthio- oder Alkylcarbamoylgruppe bedeutet, mit der Maßgabe, daß dann, wenn zwei Gruppen $R_4$ an das gleiche Kohlenstoffatom gebunden sind, diese Gruppen nicht beide Hydroxyl, Mercaptan oder Alkylthiosind; und

$R_5$ ein Wasserstoffatom oder eine Hydroxyl-, Alkoxy-, Alkylsulfonyl-, Alkyl-, Halogenalkyl-, Amino-, Alkylureido-, Alkylcarbonyloxyalkyl- oder Alkylcarbamoylgruppe bedeutet, wobei eine Alkyl- oder Halogenalkylgruppe bis zu 6 Kohlenstoffatome aufweist, mit der Maßgabe, daß dann, wenn $R_1$ eine Trifluormethylgruppe darstellt, $R_2$ ein Chloratom bedeutet, $R_3$ ein Wasserstoffatom ist, X für N—$CH_3$ steht und Z für N → O steht, Y zusätzlich für C—CH=$NCH_3$ stehen kann, zusammen mit wenigstens zwei Trägern, von denen wenigstens einer ein oberflächenaktives Mittel ist.

11. Verfahren zur Bekämpfung von unerwünschtem Pflanzenwuchs an einem Ort, welches ein Behandeln des Ortes mit einer Verbindung, wie in einem der Ansprüche 1 bis 4 beansprucht, oder mit einer Zusammensetzung, wie in Anspruch 9 oder 10 beansprucht, umfaßt.

12. Verwendung einer Verbindung, wie in einem der Ansprüche 1 bis 4 beansprucht, als Herbizid.

**Patentansprüche für den Vertragsstaat: AT**

1. Herbizide Zusammensetzung, welche eine Verbindung der allgemeinen Formel I:

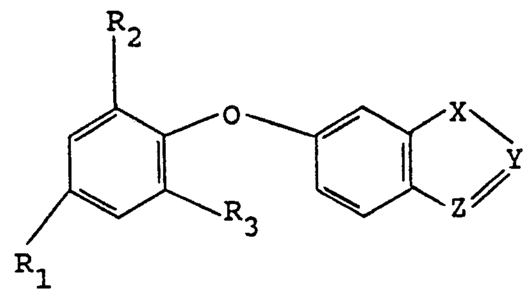

I

umfaßt, worin

$R_1$ ein Halogenatom oder eine Halogenalkylgruppe darstellt;

$R_2$ und $R_3$ die gleich oder verschieden sein können, jeweils unabhängig voneinander ein Wasserstoff- oder Halogenatom oder eine Nitro- oder Halogenalkylgruppe bedeuten;

einer der Reste von Y und Z ein Stickstoffatom oder ein oxid hievon darstellt und der andere Rest ebenfalls ein Stickstoffatom oder Oxid hievon bedeutet oder in alternativer Weise die Gruppe $CR_4$ bedeutet;

X ein Sauerstoff- oder Schwefelatom, eine Carbonyl-, Thiocarbonyl- oder Sulfonylgruppe oder eine Gruppe $NR_5$ oder $C(R_4)_2$ bedeutet; $R_4$ ein Wasserstoffatom oder eine Mercaptan-, Hydroxyl-, Alkoxy-, Alkylthio-, Alkylsulfonyl-, Alkyl-, Amino-, Alkylureido-, Alkylcarbonyloxyalkyl-, Alkoxycarbonylalkylthio- oder Alkylcarbamoylgruppe bedeutet, mit der Maßgabe, daß dann, wenn zwei Gruppen $R_4$ an das gleiche Kohlenstoffatom gebunden sind, diese Gruppen nicht beide Hydroxyl, Mercaptan oder Alkylthio sind; und

$R_5$ ein Wasserstoffatom oder eine Hydroxyl-, Alkoxy-, Alkylsulfonyl-, Alkyl-, Halogenalkyl-, Amino-, Alkylureido-, Alkylcarbonyloxyalkyl- oder Alkylcarbamoylgruppe bedeutet, wobei eine Alkyl- oder Halogenalkylgruppe bis zu 6 Kohlenstoffatome aufweist, mit der Maßgabe, daß dann, wenn $R_1$ eine Trifluormethylgruppe darstellt, $R_2$ ein Chloratom bedeutet, $R_3$ ein Wasserstoffatom ist, X für N—$CH_3$ steht und Z für N → O steht, Y zusätzlich für C—CH=$NCH_3$ stehen kann, zusammen mit wenigstens zwei Trägern, von denen wenigstens einer ein oberflächenaktives Mittel ist.

2. Zusammensetzung nach Anspruch 1, worin $R_1$ eine Trifluormethylgruppe bedeutet; $R_2$ ein Chloratom oder eine Nitrogruppe darstellt und $R_3$ ein Wasserstoffatom bedeutet.

3. Zusammensetsung nach Anspruch 1 oder 2, worin Z ein Stickstoffatom oder ein Oxid hievon bedeutet und X für ein Sauerstoff- oder Schwefelatom oder eine Gruppe $NR_5$ steht, worin $R_5$ ein Wasserstoffatom oder eine Alkyl- oder Alkylcarbamoylgruppe darstellt.

4. Zusammensetzung nach Anspruch 1, 2 oder 3, worin Y ein Stickstoffatom oder ein Oxid hievon bedeutet oder für die Gruppe $CR_4$ steht, worin $R_4$ ein Wasserstoffatom oder eine Amino- oder Mercaptangruppe oder eine Alkylthio-, Alkylsulfonyl-, Alkoxy-, Alkyl-, Alkoxycarbonylalkylthio-, Alkylcarbonyloxyalkyl- oder Alkylureidogruppe bedeutet, worin jeder Alkylrest 1 bis 4 Kohlenstoffatome enthält.

5. Verfahren zur Herstellung einer Verbindung der allgemeinen Formel I gemäß Definition in Anspruch 1, worin $R_1$ eine Halogenalkylgruppe bedeutet und $R_2$ und $R_3$ wie in Anspruch 1 definiert sind, X für Schwefel steht und Y und Z beide Stickstoffatome bedeuten, welches ein Umsetzen einer Verbindung der Formel II:

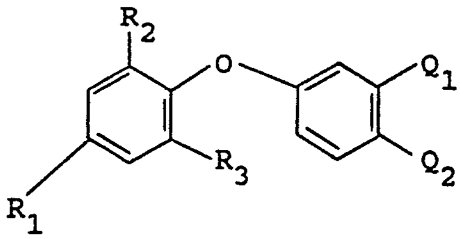

II

worin $Q_1$ eine geschützte Mercaptogruppe darstellt und $Q_2$ eine Aminogruppe bedeutet, mit einer wäßrigen Lösung eines Alkalimetallnitrits in Anwesenheit einer Säure bei einer 10°C nicht überschreitenden Temperatur umfaßt.

6. Verfahren zur Herstellung einer Verbindung der allgemeinen Formel I gemäß Definition in Anspruch 1, worin $R_1$ eine Halogenalkylgruppe bedeutet und $R_2$ und $R_3$ wie in Anspruch 1 definiert sind, X für Sauerstoff steht, Y die Gruppe CH bedeutet und Z ein Stickstoffatom darstellt, welches Verfahren ein Umsetzen einer Verbindung der Formel II, worin $Q_1$ eine Hydroxylgruppe darstellt und $Q_2$ eine Aminogruppe bedeutet, mit einem Orthoformiat umfaßt.

7. Verfahren zur Herstellung einer Verbindung der allgemeinen Formel I gemäß Definition in Anspruch 1, worin $R_1$ eine Halogenalkylgruppe darstellt, $R_2$ und $R_3$ wie in Anspruch 1 definiert sind, X ein Schwefelatom bedeutet, Y für die Gruppe $CR_4$ steht und Z ein Stickstoffatom darstellt, welches Verfahren ein Umsetzen einer Verbindung der Formel II, worin $Q_1$ eine Acylthiogruppe darstellt und $Q_2$ eine Nitrogruppe bedeutet, mit Zinn(II)-chlorid umfaßt.

8. Verfahren zur Herstellung einer Verbindung der allgemeinen Formel I gemäß Definition in Anspruch 1, worin $R_1$ eine Halogenalkylgruppe darstellt, $R_2$ und $R_3$ wie in Anspruch 1 definiert sind, X für NH steht und Y und Z beide Stickstoff bedeuten, welches Verfahren ein Umsetzen einer Verbindung der Formel II, worin $Q_1$ und $Q_2$ beide eine Aminogruppe bedeuten, mit einem Alkalimetallnitrit in Anwesenheit einer Säure umfaßt.

9. Verfahren zur Bekämpfung von unerwünschtem Pflanzenwuchs an einem Ort, welches ein Behandeln des Ortes mit einer Verbindung, wie in einem der Anspruche 1 bis 4 definiert, umfaßt.

10. Verwendung einer Verbindung, wie in einem der Ansprüche 1 bis 4 definiert, als Herbizid.

**Revendications pour les Etats contractants: BE CH DE FR GB IT LU NL SE**

1. Esters diphényliques ayant la formule générale I:

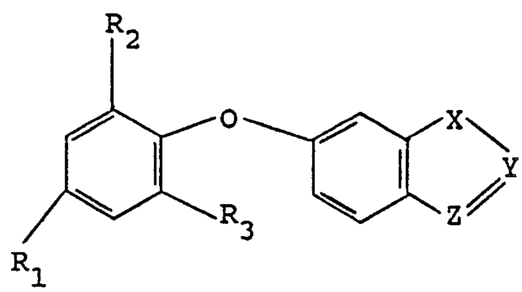

I

où

$R_1$ représente un groupe haloalcoyle;

$R_2$ et $R_3$, qui peuvent être identiques ou différents, représentent chacun indépendamment un atome d'hydrogène ou d'halogène ou un groupe nitro ou haloalcoyle;

un de Y et Z représente un atome d'azote ou un oxyde d'azote et l'autre représente aussi un atome d'azote ou un oxyde d'azote ou en variante représente le groupe $CR_4$;

X représente un atome d'oxygène ou de soufre, un groupe carbonyle, thiocarbonyle ou sulfonyle ou un groupe $NR_5$ ou $C(R_4)_2$;

$R_4$ représente un atome d'hydrogène ou un groupe mercaptan, hydroxyle, alcoxy, alcoylthio, alcoylsulfonyle, alcoyle, amino, alcoyluréido, alcoylcarbonyloxyalcoyle, alcoxycarbonylalcoylthio ou alcoylcarbamoyle avec la condition que quand deux groupes $R_4$ sont attachés au même atome de carbone, alors ces groupes ne sont pas tous deux des groupes hydroxyle, mercaptan ou alcoylthio;

et $R_5$ représente un atome d'hydrogène ou un groupe hydroxyle, alcoxy, alcoylsulfonyle, alcoyle, haloalcoyle, amino, alcoyluréido, alcoylcarbonyloxyalcoyle ou alcoylcarbamoyle, tout groupe alcoyle ou haloalcoyle ayant jusqu'à 6 atomes de carbone, avec la condition que quand $R_1$ représente un groupe trifluorométhyle, $R_2$ représente un atome de chlore, $R_3$ représente un atome d'hydrogène, X représente $N—CH_3$ et Z représente $N \rightarrow O$, Y peut en outre représenter $C—CH{=}NCH_3$.

2. Composés selon la revendication 1, dans lesquels $R_1$ est un groupe trifluorométhyle, $R_2$ est un atome de chlore ou un groupe nitro et $R_3$ est un atome d'hydrogène.

3. Composés selon la revendication 1 ou 2, dans lesquels Z représente un atome d'azote ou un oxyde d'azote et X représente un atome d'oxygène ou de soufre ou le groupe $NR_5$ représente un atome d'hydrogène ou un roupe alcoyle ou alcoylcarbamoyle.

4. Composés selon la revendication 1, 2 ou 3, dans lesquels Y représente un atome d'azote ou un oxyde d'azote, ou le groupe $CR_4$ où $R_4$ représente un atome d'hydrogène ou un groupe amino ou mercaptan ou un groupe alcoylthio, alcoylsulfonyle, alcoxy, alcoyle, alcoxycarbonylalcoylthio, alcoylcarbonyloxyalcoyle ou alcoyluréido, où chaque portion alcoyle contient 1 à 4 atomes de carbone.

5. Procédé pour la préparation d'un composé de la formule générale I où $R_1$, $R_2$ et $R_3$ sont tels que définis dans la revendication 1, X est un atome de soufre et Y et Z sont tous deux des atomes d'azote, qui comprend la réaction d'un composé de formule II:

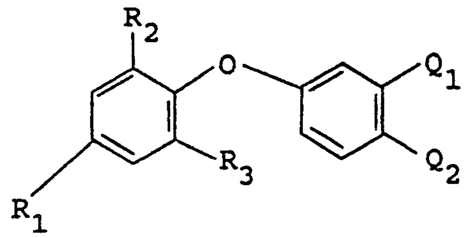

II

où $Q_1$ représente un atome mercapto protégé et $Q_2$ représente un groupe amino, avec une solution aqueuse d'un nitrite de métal alcalin en présence d'un acide à une température ne dépassant pas 10°C.

6. Procédé pour la préparation d'un composé de la formule générale I où $R_1$, $R_2$ et $R_3$ sont tels que définis dans la revendication 1, X est de l'oxygène, Y est le groupe CH et Z est un atome d'azote, qui comprend la réaction d'un composé de formule II où $Q_1$ représente un groupe hydroxyle et $Q_2$ représente un groupe amino avec un orthoformiate.

7. Procédé pour la préparation d'un composé de la formule générale I où $R_1$, $R_2$ et $R_3$ sont tels que définis dans la revendication 1, X est un atome de soufre, Y est le groupe $CR_4$ et Z est un atome d'azote, qui comprend la réaction d'un composé de formule II où $Q_1$ représente un groupe acylthio et $Q_2$ représente un groupe nitro avec du chlorure stanneux.

8. Procédé pour la préparation d'un composé de la formule générale I où $R_1$, $R_2$ et $R_3$ sont tels que définis dans la revendication 1, X est NH et Y et Z sont tous deux de l'azote, qui comprend la réaction d'un composé de formule II où $Q_1$ et $Q_2$ représentent chacun un groupe amino avec un nitrite de métal alcalin en présence d'un acide.

9. Composition herbicide, qui comprend un composé selon l'une quelconque des revendications 1 à 4, en même temps qu'un véhicule.

10. Composition herbicide, qui comprend un composé de la formule générale I:

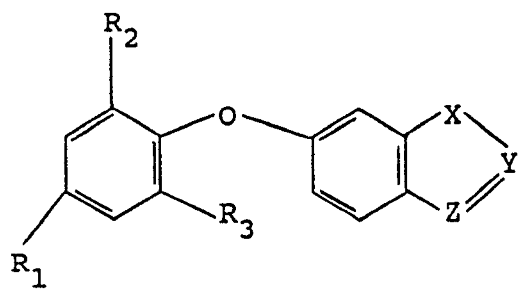

I

où

$R_1$ représente un atome d'halogène ou un groupe haloalcoyle;

$R_2$ et $R_3$, qui peuvent être identiques ou différents, représentent chacun indépendamment un atome d'hydrogène ou d'halogène ou un groupe nitro ou haloalcoyle;

un de Y et Z représente un atome d'azote ou un oxyde d'azote et l'autre représente aussi un atome d'azote ou un oxyde d'azote ou en variante représente le groupe $CR_4$;

X représente un atome d'oxygène ou de soufre, un groupe carbonyle, thiocarbonyle ou sulfonyle ou un groupe $NR_5$ ou $C(R_4)_2$;

$R_4$ représente un atome d'hydrogène ou un groupe mercaptan, hydroxyle, alcoxy, alcoylthio, alcoylsulfonyle, alcoyle, amino, alcoyluréido, alcoylcarbonyloxyalcoyle, alcoxycarbonylalcoylthio ou alcoylcarbamoyle, avec la condition que quand deux groupes $R_4$ sont attachés au même atome de carbone, alors ces groupes ne sont pas tous deux des groupes hydroxyle, mercaptan ou alcoylthio;

et $R_5$ représente un atome d'hydrogène ou un groupe hydroxyle, alcoxy, alcoylsulfonyle, alcoyle, haloalcoyle, amino, alcoyluréido, alcoylcarbonyloxyalcoyle ou alcoylcarbamoyle, tout groupe alcoyle ou haloalcoyle ayant jusqu'à 6 atomes de carbone, avec la condition que quand $R_1$ représente un groupe trifluorométhyle, $R_2$ représente un atome de chlore, $R_3$ représente un atome d'hydrogène, X représente $N—CH_3$ et Z représente $N \rightarrow O$, Y peut en outre représenter $C—CH=NCH_3$, avec en même temps au moins

deux véhicules, dont au moins un est un agent tensio-actif.

11. Procédé de lutte contre la croissance de plantes indésirables en un lieu, qui comprend le traitement du lieu avec un composé selon l'une quelconque des revendications 1 à 4 ou une composition selon la revendication 9 ou 10.

12. L'utilisation d'un composé selon l'une quelconque des revendications 1 à 4 comme herbicide.

**Revendications pour l'Etat contractant: AT**

1. Composition herbicide, qui comprend un composé de la formule générale I:

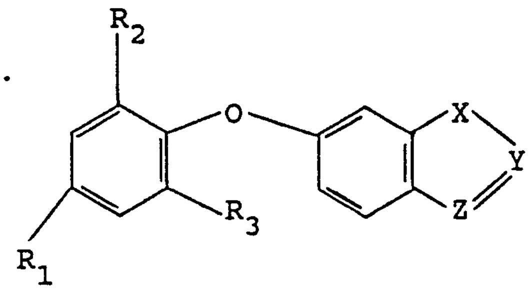

I

où

$R_1$ représente un atome d'halogène ou un groupe haloalcoyle;

$R_2$ et $R_3$, qui peuvent être identiques ou différents, représentent chacun indépendamment un atome d'hydrogène ou d'halogène ou un groupe nitro ou haloalcoyle;

un de Y et Z représente un atome d'azote ou un oxyde d'azote et l'autre représente aussi un atome d'azote ou un oxyde d'azote ou en variante représente le groupe $CR_4$;

X représente un atome d'oxygène ou de soufre, un groupe carbonyle, thiocarbonyle ou sulfonyle ou un groupe $NR_5$ ou $C(R_4)_2$;

$R_4$ représente un atome d'hydrogène ou un groupe mercaptan, hydroxyle, alcoxy, alcoylthio, alcoylsulfonyle, alcoyle, amino, alcoylthio, alcoylsulfonyle, alcoyle, amino, alcoyluréido, alcoylcarbonyloxyalcoyle, alcoxycarbonylalcoylthio ou alcoylcarbamoyle avec la condition que quand deux groupes $R_4$ sont attachés au même atome de carbone, alors ces groupes ne sont pas tous deux des groupes hydroxyle, mercaptan ou alcoylthio;

et $R_5$ représente un atome d'hydrogène ou un groupe hydroxyle, alcoxy, alcoylsulfonyle, alcoyle, haloalcoyle, amino, alcoyluréido, alcoylcarbonyloxyalcoyle ou alcoylcarbamoyle, tout groupe alcoyle ou haloalcoyle ayant jusqu'à 6 atomes de carbone, avec la condition que quand $R_1$ représente un groupe trifluorométhyle, $R_2$ représente un atome de chlore, $R_3$ représente un atome d'hydrogène, X représente $N—CH_3$ et Z représente $N \rightarrow O$, Y peut en outre représenter $C—CH=NCH_3$, avec en même temps au moins deux véhicules, dont au moins un est un agent tensio-actif.

2. Une composotion selon la revendication 1, dans lesquels $R_1$ est un groupe trifluorométhyle, $R_2$ est un atome de chlore ou un groupe nitro et $R_3$ est un atome d'hydrogène.

3. Une composition selon la revendication 1 ou 2, dans lesquels Z représente un atome d'azote ou un oxyde d'azote et X représente un atome d'oxygène ou de soufre ou le groupe $NR_5$ représente un atome d'hydrogène ou un roupe alcoyle ou alcoylcarbamoyle.

4. Une composition selon la revendication 1, 2 ou 3, dans lesquels Y représente un atome d'azote ou un oxyde d'azote, ou le groupe $CR_4$ où $R_4$ représente un atome d'hydrogène ou un groupe amino ou mercaptan ou un groupe alcoylthio, alcoylsulfonyle, alcoxy, alcoyle, alcoxycarbonylalcoylthio, alcoylcarbonyloxyalcoyle ou alcoyluréido, où chaque portion alcoyle contient 1 à 4 atomes de carbone.

5. Procédé pour la préparation d'un composé de la formule générale I défini dans la revendication 1, où $R_1$ représente un groupe haloalcoyle, $R_2$ et $R_3$ sont tels que définis dans la revendication 1, X est du soufre et Y et Z sont tous deux des atomes d'azote, qui comprend la réaction d'un composé de formule II:

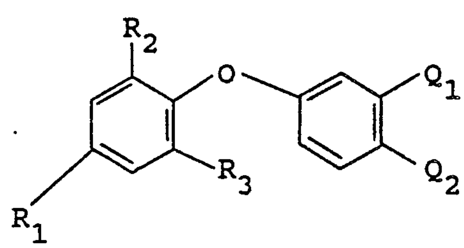

II

où $Q_1$ représente un groupe mercapto protégé et $Q_2$ représente un groupe amino, avec une solution aqueuse d'un nitrite de métal alcalin en présence d'un acide à une température ne dépassant pas 10°C.

6. Procédé pour la préparation d'un composé de la formule générale I défini dans la revendication 1 dans lequel $R_1$ représente un groupe haloalcoyle, $R_2$ et $R_3$ sont tels que définis dans la revendication 1, X est

de l'oxygène, Y est le groupe CH et Z est un atome d'azote, qui comprend la réaction d'un composé de formule II dans lequel $Q_1$ représente un groupe hydroxyle et $Q_2$ représente un groupe amino avec un orthoformiate.

7. Procédé pour la préparation d'un composé de la formule générale I défini dans la revendication 1 où $R_1$ représente un groupe haloalcoyle, $R_2$ et $R_3$ sont tels que définis dans la revendication 1, X est un atome de soufre, Y est le groupe $CR_4$ et Z est un atome d'azote qui comprend la réaction d'un composé de formule II où $Q_1$ représente un groupe acylthio et $Q_2$ représente un groupe nitro avec du chlorure stanneux.

8. Procédé pour la préparation d'un composé de la formule générale I défini dans la revendication 1 où $R_1$ représente un groupe haloalcoyle, $R_2$ et $R_3$ sont tels que définis dans la revendication 1, X est NH et Y et Z sont tous deux de l'azote, qui comprend la réaction d'un composé de formule II où $Q_1$ et $Q_2$ représentent chacun un groupe amino avec un nitrite de métal alcalin en présence d'un acide.

9. Procédé de lutte contre la croissance de plantes indésirables en un lieu, qui comprend le traitement du lieu avec un composé tel que défini dans l'une quelconque des revendications 1 à 4.

10. L'utilisation d'un composé tel que défini dans l'une quelconque des revendications 1 à 4 comme herbicide.